# EUROPEAN PATENT APPLICATION

(11) **EP 4 850 591 A1**
(43) Date of publication of application: **09.12.2026**
(21) Application number: 26179615.5
(22) Date of filing: 19.05.2026
(51) Int. Cl.: A61B 3/107, A61B 3/10

(54) **DEVICE FOR MEASURING CORNEAL CURVATURE AND DRY EYE SYNDROME**

(30) Priority: 05.06.2025 KR 20250073341
(71) Applicant: Huvitz Co., Ltd., Anyang-si, Gyeonggi-do 14055 (KR)
(72) Inventor: JO, Sun Hyung, 14055 Anyang-si (KR); JU, Ye Na, 14055 Anyang-si (KR)
(74) Representative: Henkel & Partner mbB

(57) **Abstract**

A device for measuring corneal curvature and dry eye syndrome is disclosed. The device (10) for measuring corneal curvature and dry eye syndrome includes: a measurement light illumination unit (100) for irradiating corneal curvature measurement light having a predetermined pattern onto an eye under examination (5), wherein a through-hole (120) for observing an image of the eye under examination (5) is formed in a center thereof; a wide-angle lens (200) mounted in the through-hole (120) of the measurement light illumination unit (100) to diffuse and transmit the image of the eye under examination (5); a magnification changing unit (300) for adjusting a magnification of the image of the eye under examination (5) observed through the wide-angle lens (200); and an image detection unit (500) for detecting the image of the eye under examination (5) transmitted through the magnification changing unit (300) to obtain corneal curvature and dry eye syndrome diagnosis information of the eye under examination (5).

## Description

### CROSS-REFERENCE TO RELATED APPLICATION

This application claims benefit of priority to Korean Patent Application No. 10-2025-0073341 filed on June 05, 2025, the entire contents of which are incorporated herein by reference.

### FIELD OF THE DISCLOSURE

The present invention relates to an optometry device, and more particularly, to a device capable of measuring both corneal curvature and dry eye syndrome.

### BACKGROUND

Dry eye syndrome is an ocular disease in which the ocular surface is damaged due to a lack of tears or an imbalance in the components of the tear film, causing stimulatory symptoms such as stinging, irritation, foreign body sensation, and dryness.

The tear film consists of a mucus layer, an aqueous layer, and a lipid layer. If abnormalities occur in these layers, dry eye syndrome may occur. For example, meibomian gland dysfunction, which secretes oil, or blepharitis can be causes of dry eye syndrome. Additionally, looking at digital devices for a long time can lead to dry eye syndrome as the frequency of blinking decreases. When the ocular surface dries due to dry eye syndrome, the protective layer against external foreign substances disappears, which may lead to diseases such as corneal inflammation, corneal ulcers, and allergies.

Currently, methods for diagnosing dry eye syndrome include a tear production test, a tear film test, and staining to determine the state of corneal epithelial cells. In a tear film examining method using an optometry device, the eye under examination is illuminated with illumination light, an image of the eye illuminated with the illumination light is detected, and whether there is an abnormality in the tear layer formed on the corneal surface of the eye is examined (see Patent Documents 1 to 3).

Meanwhile, a corneal topography device is also known which measures the corneal curvature of an eye under examination by irradiating infrared measurement light in the form of Placido rings onto the cornea of the eye under examination and analyzing the shape of the Placido ring images formed on the eye under examination.

### [Prior Art Documents]

(Patent Document 1) Korean Patent No. 10-1471249 (Dec. 03, 2014)
(Patent Document 2) Korean Patent No. 10-2728315 (Nov. 05, 2024)
(Patent Document 3) Korean Patent No. 10-2293429 (Aug. 19, 2021)

### SUMMARY OF THE DISCLOSURE

### TECHNICAL OBJECTS

An object of the present invention is to provide a device capable of measuring both corneal curvature and dry eye syndrome.

Another object of the present invention is to provide a measuring device capable of efficiently examining corneal curvature and dry eye syndrome by increasing the image detection area of an eye under examination illuminated with illumination light while increasing the irradiation area of measurement light for measuring corneal curvature.

### TECHNICAL SOLUTION

In order to achieve the above objects, the present invention provides a device for measuring corneal curvature and dry eye syndrome, comprising: a measurement light illumination unit for irradiating corneal curvature measurement light having a predetermined pattern onto an eye under examination, wherein a through-hole for observing an image of the eye under examination is formed in a center thereof; a wide-angle lens mounted in the through-hole of the measurement light illumination unit to diffuse and transmit the image of the eye under examination; a magnification changing unit for adjusting a magnification of the image of the eye under examination observed through the wide-angle lens; and an image detection unit for detecting the image of the eye under examination transmitted through the magnification changing unit to obtain corneal curvature and dry eye syndrome diagnosis information of the eye under examination.

Here, the wide-angle lens may be a meniscus lens having a concave front surface toward the eye under examination.

The measurement light illumination unit may include: a light emitting diode for emitting measurement light; a hemispherical reflecting plate for reflecting the measurement light emitted from the light emitting diode; and a Placido ring plate in which a plurality of measurement light transmission rings having the same center are formed to transmit the measurement light reflected from the reflecting plate as a plurality of ring-shaped lights having the same center.

The magnification changing unit may increase or decrease the magnification of the image of the eye under examination by inserting or removing an enlargement lens for enlarging the magnification of the image of the eye under examination or by inserting or removing a reduction lens for reducing the magnification of the image of the eye under examination on an optical path for acquiring the image of the eye under examination.

In addition, the dry eye syndrome diagnosis information may be obtained by detecting a composition of a tear film or an image of a meibomian gland from the image of the eye under examination.

### EFFECTS OF THE DISCLOSURE

According to the device for measuring corneal curvature and dry eye syndrome according to the present invention, by increasing the image detection area of the eye under examination illuminated with illumination light while increasing the irradiation area of the measurement light for measuring corneal curvature, the corneal curvature and dry eye syndrome can be efficiently examined.

### BRIEF DESCRIPTION OF THE DRAWINGS

FIG. 1 is a view for explaining a configuration of a device for measuring corneal curvature and dry eye syndrome according to an embodiment of the present invention.
FIG. 2 shows a plan view and a side cross-sectional view of a measurement light illumination unit that can be used in the present invention.
FIG. 3 is a view for explaining that the magnification of an image of an eye under examination is adjusted in the device for measuring corneal curvature and dry eye syndrome according to an embodiment of the present invention.
FIG. 4 is a view showing an example of a magnification changing unit for adjusting the magnification of an image of an eye under examination in the device for measuring corneal curvature and dry eye syndrome according to an embodiment of the present invention.

### DETAILED DESCRIPTION OF THE PREFERRED EMBODIMENTS

Hereinafter, the present invention will be described in detail with reference to the accompanying drawings.

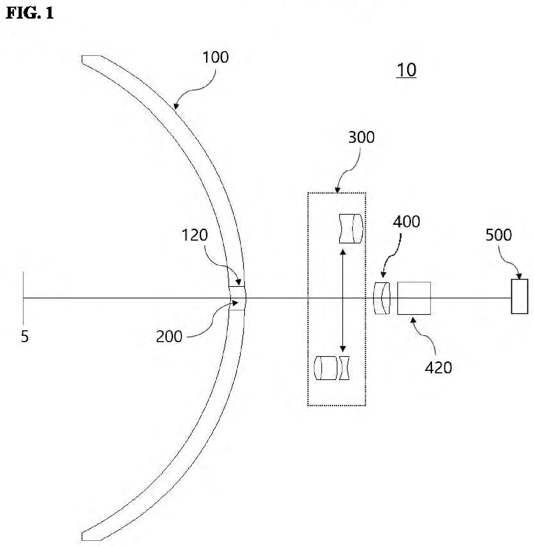

FIG. 1 is a view for explaining a configuration of a device for measuring corneal curvature and dry eye syndrome according to an embodiment of the present invention. As shown in FIG. 1, the device 10 for measuring (in other word, determining) corneal curvature and dry eye syndrome according to the present invention includes a measurement light illumination unit 100, a wide-angle lens 200, a magnification changing unit 300, and an image detection unit 500.

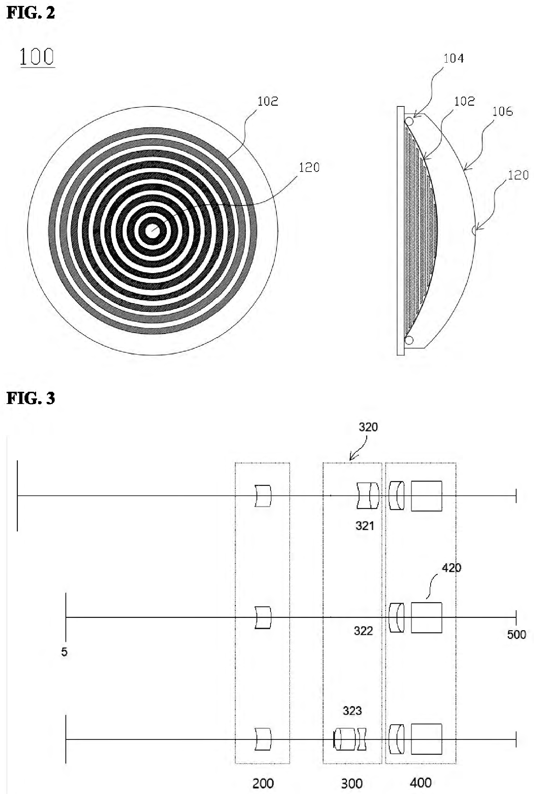

The measurement light illumination unit 100 is a device for irradiating corneal curvature measurement light having a predetermined pattern onto an eye under examination 5. FIG. 2 shows a plan view and a side cross-sectional view of the measurement light illumination unit 100 that can be used in the present invention. As shown in FIG. 2, the measurement light illumination unit 100 that can be used in the present invention may include a light emitting diode 104 that emits measurement light, for example, infrared light, a hemispherical reflecting plate 106 that reflects the measurement light emitted from the light emitting diode 104, and a Placido ring plate 102 in which a plurality of measurement light transmission rings having the same center are formed to transmit the measurement light reflected from the reflecting plate 106 as a plurality of ring-shaped lights having the same center. The light emitting diode 104 is positioned in a donut shape at the edge of the reflecting plate 106. Accordingly, the measurement light illumination unit 100 can emit measurement light in the form of Placido rings toward the eye under examination 5.

The measurement light emitted from the measurement light illumination unit 100 is irradiated onto the cornea of the eye under examination 5. At this time, it is preferable that the corneal vertex of the eye under examination 5 is located on the central axis of the measurement light, i.e., the Placido rings. The pattern of the measurement light irradiated onto the cornea of the eye under examination 5, i.e., the image of the Placido rings, is deformed according to the corneal curvature of the eye under examination 5. Therefore, if the pattern image of the measurement light irradiated onto the cornea of the eye under examination 5 is detected and analyzed, corneal curvature information of the eye under examination 5 can be obtained.

The measurement light illumination unit 100 may also illuminate the eye under examination 5 with visible light in order to examine dry eye syndrome. For example, dry eye syndrome can be diagnosed by illuminating the eye under examination 5 with white light from the measurement light illumination unit 100, detecting an image of the eye under examination 5 illuminated with the white light using the image detection unit 500, and then analyzing the composition of the tear film, such as the thickness of the lipid layer, or the image of the meibomian gland from the detected image of the eye under examination 5. Although the measurement light illumination unit 100 may be used as a light source for illuminating the eye under examination 5 to examine dry eye syndrome, a separate light source may also be used instead of the measurement light illumination unit 100.

As shown in FIGs. 1 and 2, a through-hole 120 for observing the eye under examination 5 is formed in the center of the measurement light illumination unit 100. For example, as shown in FIG. 2, the through-hole 120 is formed in the center of the reflecting plate 106 and the Placido ring plate 102. Through the through-hole 120, an image of the eye under examination 5 to which the measurement light is irradiated is obtained, and corneal curvature information of the eye under examination 5 can be obtained by analyzing the pattern of the measurement light formed on the eye under examination 5.

The diameter of the through-hole 120 may be about 5 to 11 mm, for example, 7 to 9 mm. If the diameter of the through-hole 120 is large, an image of the eye under examination 5 can be obtained in a wider area with high resolution, but the Placido ring formation area of the measurement light illumination unit 100 becomes smaller, and the obtained corneal curvature information decreases. Conversely, if the diameter of the through-hole 120 is small, the Placido ring formation area increases, so the obtained corneal curvature information increases, but the image of the eye under examination 5 must be obtained through the small through-hole 120, and the observation area and the resolution of the image of the eye under examination 5 decrease.

The wide-angle lens 200 is mounted in the through-hole 120 of the measurement light illumination unit 100, specifically in the Placido ring plate 102, and diffuses and transmits the image of the eye under examination 5. The wide-angle lens 200 may be a meniscus lens having a concave front surface 210 toward the eye under examination 5 (see FIG. 3). The wide-angle lens 200 is a wide field-of-view lens that allows a wider area image of the eye under examination 5 to be observed by diverging and transmitting the image of the eye under examination 5.

The wide-angle lens 200 diffuses and transmits reflected light of the measurement light reflected from the eye under examination 5. Therefore, an image of the eye under examination 5 can be acquired in a relatively wide area even if the through-hole 120 of the measurement light illumination unit 100 is formed relatively small. If necessary, the rear surface 220 of the wide-angle lens 200 may also be formed to be convex (see FIG. 3). The diameter of the wide-angle lens 200 is the same as the diameter of the through-hole 120 and may be about 5 to 11 mm, for example, 7 to 9 mm. For example, the absolute value of the refractive power (P) of the wide-angle lens 200 is 0.01 or less and the focal length (F) thereof is 100 mm or more.

By mounting the wide-angle lens 200, the through-hole 120 can be formed relatively small, and more Placido rings are formed in the measurement light illumination unit 100 around the through-hole 120, for example, on the Placido ring plate 102, thereby increasing the number of Placido rings irradiated onto the cornea of the eye under examination 5, so that the corneal curvature of the eye under examination 5 can be measured more precisely.

The magnification changing unit 300 adjusts the magnification of the image of the eye under examination 5 observed through the wide-angle lens 200. In order to evaluate corneal curvature and dry eye syndrome from the image of the eye under examination 5, it is necessary to adjust the enlargement magnification of the image of the eye under examination 5. That is, the magnification of the image of the eye under examination 5 should be adjusted so that an object to be measured or a property to be measured can be visually and properly displayed according to the type of measurement. For example, the corneal curvature of the corneal surface can be measured using a reference magnification. The tear film, such as the thickness of the lipid layer, can be measured or analyzed using an enlargement magnification larger than the reference magnification. In addition, an image of the meibomian gland can be obtained using a reduction magnification smaller than the reference magnification.

FIG. 3 is a view for explaining that the magnification of an image of an eye under examination is adjusted in the device for measuring corneal curvature and dry eye syndrome according to an embodiment of the present invention. As shown in FIG. 3, the magnification changing unit 300 may increase or decrease the magnification of the image of the eye under examination 5 by inserting or removing an enlargement lens 321 for enlarging the magnification of the image or by inserting or removing a reduction lens 323 for reducing the magnification of the image on the optical path for acquiring the image of the eye under examination 5. The reference magnification may be implemented by not inserting a separate lens on the optical path for acquiring the image or by inserting a light-transmissive transparent plate 322 that does not enlarge or reduce the image of the eye under examination 5.

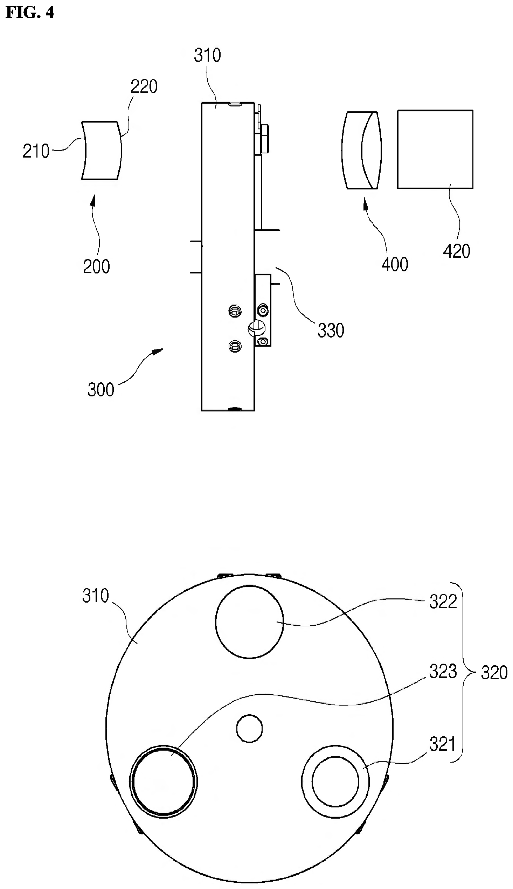

FIG. 4 is a view showing an example of the magnification changing unit 300 for adjusting the magnification of the image of the eye under examination 5. As shown in FIGS. 3 and 4, the magnification changing unit 300 includes a magnification changing lens unit 320 including the enlargement lens 321, the reduction lens 323, and the light-transmissive transparent plate 322. More specifically, the magnification changing unit 300 may include a rotating body 310 in which the enlargement lens 321, the reduction lens 323, and the light-transmissive transparent plate 322 are mounted at predetermined angular intervals, and an alignment driving unit 330 that rotates the rotating body 310 to position any one of the enlargement lens 321, the reduction lens 323, and the light-transmissive transparent plate 322 on the optical path where the image of the eye under examination 5 is obtained. For example, the enlargement lens 321, the reduction lens 323, and the light-transmissive transparent plate 322 are installed at uniform angles relative to each other in a circumferential direction based on a center point of the circular rotating body 310, and the alignment driving unit 330 rotates a rotation axis formed in a center of the rotating body 310 or rotates an outer peripheral surface of the rotating body 310.

The image detection unit 500 detects the image of the eye under examination 5 transmitted through the magnification changing unit 300 to obtain corneal curvature and dry eye syndrome diagnosis information. The image detection unit 500 includes an image sensor that detects reflected light reflected from the eye under examination 5 to obtain an image of the eye under examination 5. For example, a conventional charge-coupled device (CCD) camera may be used as the image sensor.

The image of the eye under examination 5 detected by the image sensor is analyzed in the image detection unit 500, specifically in a calculation unit (not shown) of the image detection unit 500. Thereby, corneal shape information such as corneal curvature can be obtained. Also, dry eye syndrome diagnosis information can be obtained from the composition of the tear film, such as the thickness of the lipid layer, or the image of the meibomian gland. That is, the dry eye syndrome diagnosis information can be obtained by detecting the composition of the tear film (for example, thicknesses of layers) or the image of the meibomian gland from the image of the eye under examination 5.

As shown in FIGS. 1, 3, and 4, conventional optical elements such as an objective lens 400 for focusing the image of the eye under examination 5 and transmitting it to the image detection unit 500, and a beam splitter 420 for reflecting and transmitting fixation light, glare light, etc., to the eye under examination 5 may further be positioned on the optical path where the image of the eye under examination 5 is obtained.

According to the present invention, by mounting the wide-angle lens 200 in the through-hole 120 of the measurement light illumination unit 100, the through-hole 120 can be formed relatively small, and more Placido rings can be formed in the measurement light illumination unit 100 around the through-hole 120, for example, on the Placido ring plate 102, thereby increasing the number of Placido rings irradiated onto the cornea of the eye under examination 5, so that the corneal curvature of the eye under examination 5 can be measured more precisely.

The present invention has been described above with reference to the accompanying drawings and exemplary embodiments, but the present invention is not limited to the contents shown in the drawings and the above-described embodiments. Although reference numerals are provided in the following claims for easy understanding, the scope of the following claims is not limited to the reference numerals and the contents shown in the drawings, but should be interpreted to encompass all modifications, equivalent configurations, and functions of the exemplary embodiments.

## Claims

1. A device (10) for measuring corneal curvature and dry eye syndrome, comprising:
a measurement light illumination unit (100) for irradiating corneal curvature measurement light having a predetermined pattern onto an eye under examination (5), wherein a through-hole (120) for observing the eye under examination (5) is formed in a center thereof;
a wide-angle lens (200) mounted in the through-hole (120) of the measurement light illumination unit (100) to diffuse and transmit the image of the eye under examination (5);
a magnification changing unit (300) for adjusting a magnification of the image of the eye under examination (5) observed through the wide-angle lens (200); and
an image detection unit (500) for detecting the image of the eye under examination (5) transmitted through the magnification changing unit (300) to obtain corneal curvature and dry eye syndrome diagnosis information of the eye under examination (5).

2. The device (10) for measuring corneal curvature and dry eye syndrome of claim 1, wherein the wide-angle lens (200) is a meniscus lens having a concave front surface (210) toward the eye under examination (5).

3. The device (10) for measuring corneal curvature and dry eye syndrome of claim 1, wherein the measurement light illumination unit (100) comprises: a light emitting diode (104) for emitting measurement light; a hemispherical reflecting plate (106) for reflecting the measurement light emitted from the light emitting diode (104); and a Placido ring plate (102) in which a plurality of measurement light transmission rings having the same center are formed to transmit the measurement light reflected from the reflecting plate (106) as a plurality of ring-shaped lights having the same center.

4. The device (10) for measuring corneal curvature and dry eye syndrome of claim 1, wherein the magnification changing unit (300) increases or decreases the magnification of the image of the eye under examination (5) by inserting or removing an enlargement lens (321) for enlarging the magnification of the image or by inserting or removing a reduction lens (323) for reducing the magnification of the image on an optical path for acquiring the image of the eye under examination (5).

5. The device (10) for measuring corneal curvature and dry eye syndrome of claim 1, wherein the dry eye syndrome diagnosis information is obtained by detecting a composition of a tear film or an image of a meibomian gland from the image of the eye under examination (5).
